# EUROPEAN PATENT APPLICATION

(11) **EP 4 129 158 A1**
(43) Date of publication of application: **08.02.2023**
(21) Application number: 21779845.3
(22) Date of filing: 25.03.2021
(51) Int. Cl.: A61B 5/00, A61B 5/33

(54) **BIOSENSOR, BIOSENSOR SYSTEM, AND BIOSENSOR OPERATION CONTROL METHOD**

(30) Priority: 30.03.2020 JP 2020059655
(71) Applicant: Nitto Denko Corporation, Ibaraki-shi, Osaka 567-8680 (JP); Spchange, LLC, Yokohama-shi, Kanagawa 224-0032 (JP)
(72) Inventor: YOSHIOKA, Ryoma, Ibaraki-shi, Osaka 567-8680 (JP); KAMATA, Takatsugu, Yokohama-shi, Kanagawa 224-0032 (JP); HIRAI, Yusaku, Yokohama-shi, Kanagawa 224-0032 (JP); UEDA, Masayuki, Yokohama-shi, Kanagawa 224-0032 (JP)
(74) Representative: Hoffmann Eitle
(86) International application number: PCT/JP2021/012651
(87) International publication number: WO 2021/200576

(57) **Abstract**

A biosensor includes an electrode configured to be attached to a living body; an obtaining section configured to obtain biological information through the electrode; a memory configured to store the biological information obtained by the obtaining section; and a controller including an operation checking mode and a biological information recording mode. The controller, during the operation checking mode, wirelessly transmits the biological information obtained by the obtaining section, and transitions to the biological information recording mode upon receiving a recording start command from an outside, and during the biological information recording mode, writes the biological information obtained by the obtaining section to the memory. This allows to provide a biosensor having a function that enables to check whether the biological information can be obtained normally before starting the measurement of biological information.

## Description

### [Technical Field]

The present disclosure relates to a biosensor, a biosensor system and operation control method of biosensor.

### [Background Art]

A wearable biosensor that can be attached to a living body to obtain biological information such as an electrocardiographic signal over a long time is known. For example, a biosensor of this type has electrodes on both longitudinal directional sides, the electrodes are affixed to a chest of a living body with the longitudinal direction aligned with a sternum, and then, the biosensor automatically starts measuring biological information (see, for example, Patent Document 1).

### [Prior Art Documents]

### [Patent Documents]

[Patent Document 1] the specification of U.S. Patent Application Publication No. 2019/0254553

### [Summary of Invention]

### [Problem to be Solved by Invention]

When biological information is obtained for a long time by a biosensor affixed to a living body, measurement for a long time becomes useless if the biosensor cannot obtain biological information properly. In order to prevent the measurement for a long time being useless, it is preferable to check whether the biological information can be obtained normally before starting the measurement of biological information (actual measurement).

The present invention has been devised in view of the above-described points, and the present invention has an object to provide a biosensor having a function that enables to check whether the biological information can be obtained normally before starting the measurement of biological information.

### [Means for Solving Problem]

A biosensor according to an embodiment of the present invention includes an electrode configured to be attached to a living body, an obtaining section configured to obtain biological information through the electrode, a memory configured to store the biological information obtained by the obtaining section, and a controller including an operation checking mode and a biological information recording mode. The controller, during the operation checking mode, wirelessly transmits the biological information obtained by the obtaining section, and transitions to the biological information recording mode upon receiving a recording start command from an outside, and during the biological information recording mode, writes the biological information obtained by the obtaining section to the memory.

### [Advantageous Effects of Invention]

According to the disclosed technique, it is possible to provide a biosensor having a function that enables to check whether the biological information can be obtained normally before starting the measurement of biological information.

### [Brief Description of Drawings]

FIG. 1 is a diagram illustrating an example of an overall configuration of a biosensor system including a biosensor according to an embodiment;
FIG. 2 is a layout diagram illustrating an example of a flexible substrate of FIG. 1;
FIG. 3 is a diagram illustrating a state in which the biosensor of FIG. 1 is affixed to a chest of a subject;
FIG. 4 is a block diagram illustrating an example of a circuit configuration of the biosensor of FIG. 1;
FIG. 5 is a state transition diagram illustrating an example of a transition of a mode of operation of the biosensor of FIG. 1;
FIG. 6 is a flowchart illustrating an example of the operation of the biosensor of FIG. 1;
FIG. 7 is a flowchart illustrating a continuation of the operation of FIG. 6;
FIG. 8 is a flowchart illustrating a continuation of the operation of FIG. 7;
FIG. 9 is a flowchart illustrating a continuation of the operation of FIG. 8;
FIG. 10 is a sequence diagram illustrating an example of operation in a pairing mode of FIG. 5 and FIG. 7;
FIG. 11 is a data flow diagram illustrating an example of data transmission/reception between the biosensor of FIG. 1 and an operation checking device; and
FIG. 12 is an explanatory diagram illustrating a display example of a screen of a PC connected to the operation checking device of FIG. 1.

### [Description of Embodiments]

Hereinafter, embodiments of the invention will be described with reference to the drawings. In each drawing, the same components are indicated by the same reference numerals and duplicate descriptions may be omitted. The same reference numerals as the signal name or voltage name is used for the signal line or voltage line through which information such as a signal or voltage is transmitted. Further, a signal line with the reference numerals "/" indicates a plurality of bits, but a signal line indicated by a single line may also transmit a signal of a plurality of bits.

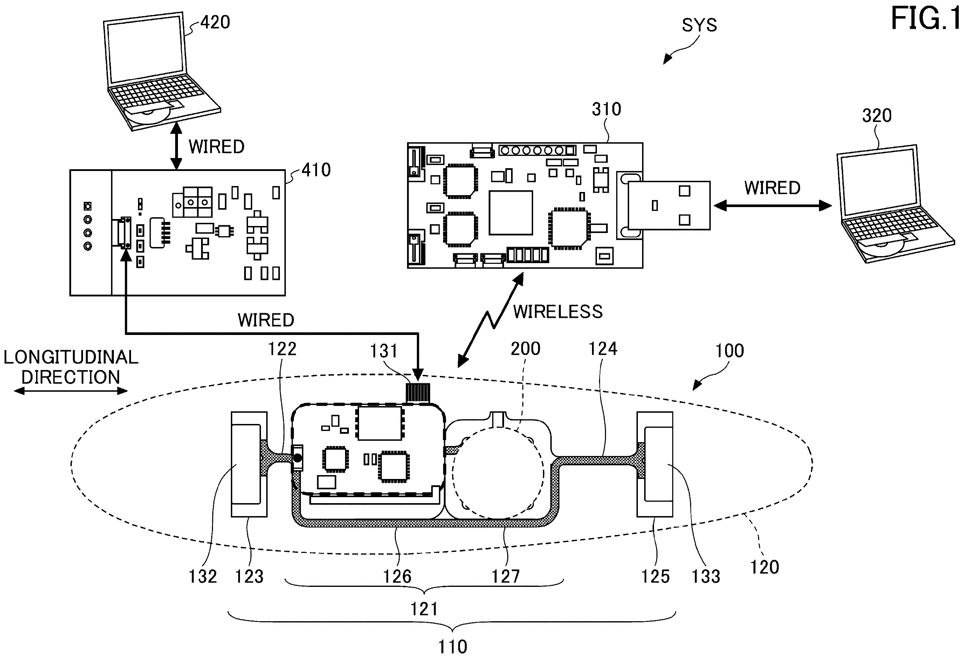

FIG. 1 is a diagram illustrating an example of an overall configuration of a biosensor system including a biosensor according to an embodiment. A biosensor system SYS of FIG. 1 includes a biosensor 100, an operation checking device 310 for checking an initial operation, a personal computer (PC) 330, a reading device 410 for reading biological information from the biosensor 100, and a PC 420. Each of the operation checking device 310 and the reading device 410 is an example of an external device.

For example, the biosensor 100 is a wearable electrocardiograph that obtains an electrocardiographic signal from a living body. The biosensor 100 may have a function of obtaining biological information other than an electrocardiographic signal, and may have a function of obtaining a plurality of types of biological information.

The operation checking device 310 is connected to the PC 320 via, for example, a universal serial bus (USB) interface (wired). The operation checking device 310 has a capability of wirelessly communicating with the biosensor 100 under the control of the PC 320. For example, the PC 320 has a function of displaying a waveform representing a time change in received biological information (for example, an electrocardiographic waveform) on a display screen.

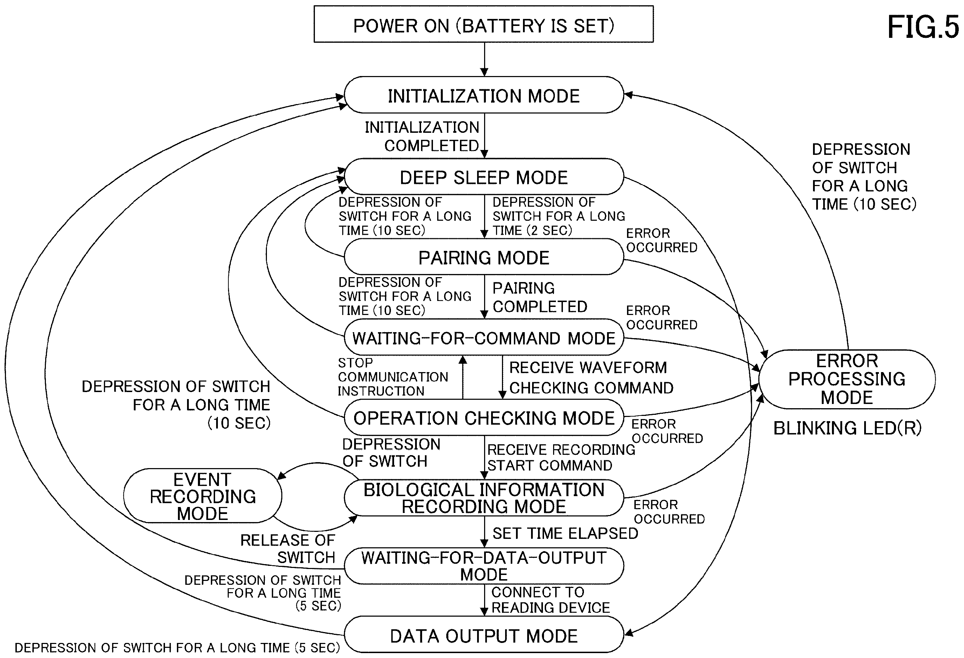

The reading device 410 is connected to the PC 420, for example, via a USB interface (wired). The reading device 410 has a function of communicating with the biosensor 100 via a communication cable (wired communication). The details of the operation of the biosensor 100, the operation checking device 310, and the reading device 410 will be described later with reference to FIG. 5.

The biosensor 100 includes a flexible substrate (a resin substrate) 110 and a housing 120 (depicted by a dashed line) on which various components are mounted for obtaining biological information and processing the obtained biological information. The flexible substrate 110 includes a body section 121, a constricted section 122 provided at one longitudinal directional end of the body section 121, and a pad section 123 connected to the body section 121 via the constricted section 122. The flexible substrate 110 also includes a constricted section 124 provided at the other longitudinal directional end of the body section 121 and a pad section 125 connected to the body section 121 via the constricted section 124.

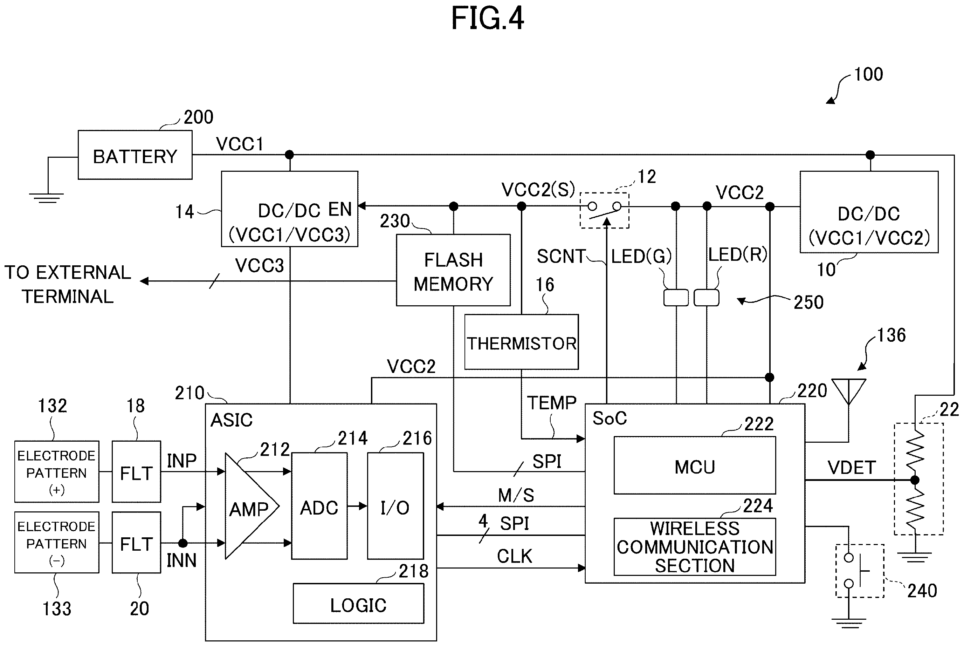

The body section 121 includes a component mounting section 126 at the constricted section 122 side and a battery setting section 127, to which a battery 200 is set, at the constricted section 124 side. Various components mounted on the component mounting section 126 and circuit examples using the various components will be described with reference to FIG. 4. An external terminal 131, to which a connector of a communication cable to be connected to the reading device 410 is connected, is formed in the component mounting section 126.

For example, a coin-type battery 200 that supplies power to the component mounting section 126 is set at the battery setting section 127. An electrode pattern 132 is formed on the pad section 123 to be affixed to a body surface of a living body, and an electrode pattern 133 is formed on the pad section 125 to be affixed to the body surface of the living body. Hereinafter, the electrode pattern 132 is also referred to as an electrode 132, and the electrode pattern 133 is also referred to as an electrode 133.

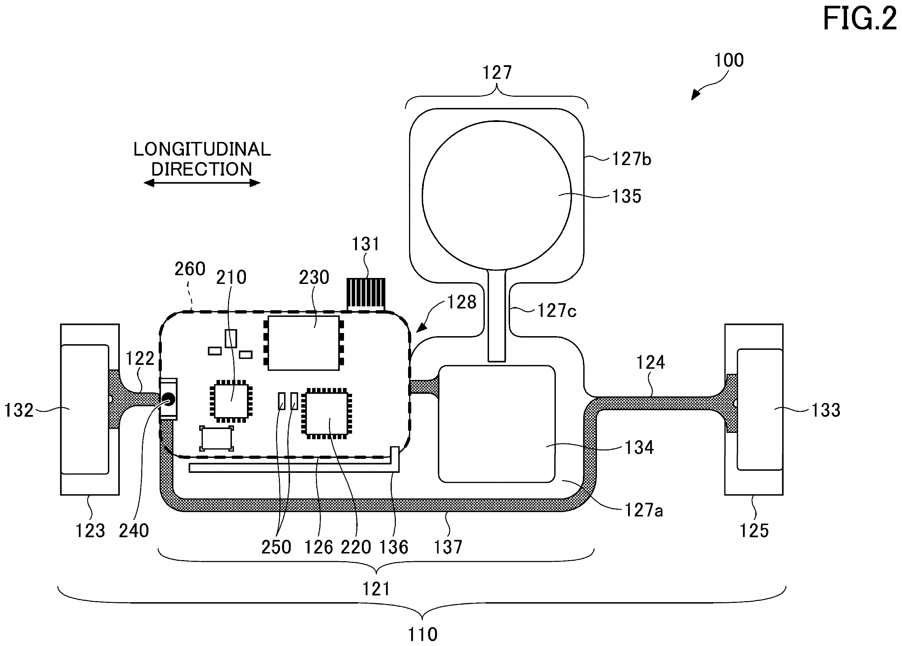

FIG. 2 is a layout diagram illustrating an example of the flexible substrate 110 of FIG. 1. On the component mounting section 126 of the flexible substrate 110, an application specific integrated circuit (ASIC) 210, a system on a chip (SoC) 220, a NAND type flash memory 230, a switch 240, and light emitting diodes (LED) 250 are mounted. As illustrated in FIG. 4, a LED (G) that outputs green light and a LED (R) that outputs red light are mounted to the component mounting section 126 as the LED 250. The LED (G) and the LED (R) are examples of light emitting elements.

The biosensor 100 includes a plate member 260 (depicted in FIG. 2 as a frame drawn by a thick broken line), such as a stainless steel plate, on a surface (back side), included in the flexible substrate 110, opposite to a component mounting surface (front side) on which the components such as the ASIC 210 and the SoC 220 are mounted. The switch 240 is, for example, a depression switch that is set to an on state when a protrusion is depressed and set to an off state when the protrusion is not depressed. The switch 240 is mounted at a position that is next to the constricted section 122 (at an edge of the body section 121) and is opposite the plate member 260. Hereinafter, a living body to which the biosensor 100 is affixed and from which biological information is obtained by the biosensor 100 is also referred to as a subject P.

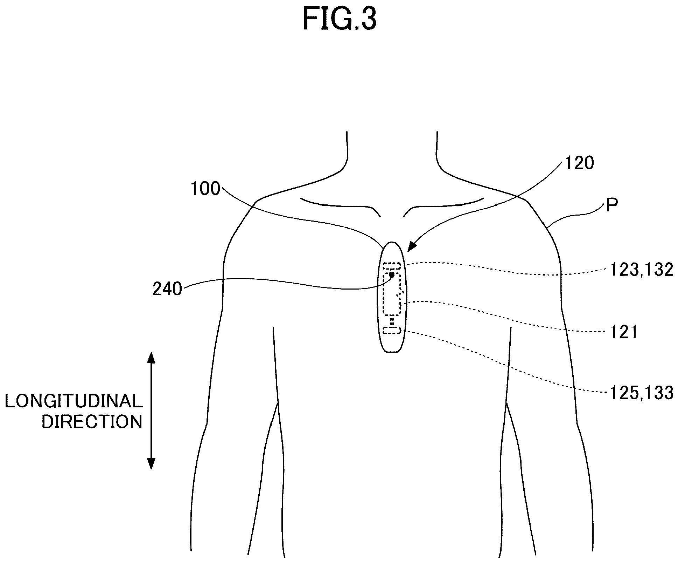

The constricted section 124 is formed to be longer than the constricted section 122. As illustrated in FIG. 3, the biosensor 100 is affixed along a sternum of the subject P, while the pad section 123 is faced upward (i.e., toward the subject P neck side). Adhesion of electrodes 132 and 133 to a body surface of the subject P may be implemented with the use of an electrically conductive adhesive, or may be implemented with the use of a non-electrically-conductive adhesive to be applied to a part of each of the electrodes 132 and 133. Alternatively, an electrode that is specially prepared for being affixed to a living body and is affixed to each of the electrodes 132 and 133 may be used to affix the electrodes 132 and 133 to the subject P with the use of an electrically conductive adhesive or a non-electrically-conductive adhesive. Note that the non-electrically-conductive adhesive is partially applied to the electrode that is specially prepared for being affixed to the living body.

The battery setting section 127 includes pad sections 127a and 127b and a constricted section 127c. The pad section 127a is provided between the constricted section 124 and the component mounting section 126. The pad section 127b is provided, in a direction orthogonal to the longitudinal direction, with respect to the pad section 127a (an upper direction of FIG. 2) at a predetermined distance from the pad section 127a. The constricted section 127c is provided between pad sections 127a and 127b to connect the pad sections 127a and 127b together.

The pad section 127a has a positive electrode pattern 134 to which a positive terminal of a battery 200 (FIG. 1) is connected. The pad section 127b has a negative electrode pattern 135 to which a negative terminal of the battery 200 is connected. For example, the positive electrode pattern 134 has a square shape with corners chamfered, and the negative electrode pattern 135 has a circular shape corresponding to a size of a circular shape of the negative terminal of the battery 200. For example, a diameter of the negative electrode pattern 135 is equal to a diameter of the battery 200 and is equal to a length of a diagonal of the positive electrode pattern 134.

When a battery 200 is set in the biosensor 100, an electrically conductive adhesive, such as an adhesive tape, is attached throughout the positive and negative electrode patterns 134 and 135. Then, the battery 200 is mounted on the battery setting portion 127 by attaching the positive electrode terminal and the negative electrode terminal of the battery 200 to the positive electrode pattern 134 and the negative electrode pattern 135, respectively, via the adhesive. The body section 121 depicted in FIG. 1 is in a state where a battery 200 is set at the battery setting section 127 with the battery 200 sandwiched between the positive electrode pattern 134 and the negative electrode pattern 135 with the constricted section 127c bent.

The flexible substrate 110 has an antenna pattern 136 formed along the longitudinal direction of the flexible substrate 110 near one (at a lower side in FIG. 2) of four sides of the rectangular component mounting section 126. Although not depicted, one end of the antenna pattern 136 is connected to the SoC 220. The flexible substrate 110 also has a wiring pattern 137 formed at an edge (at a lower side in FIG. 2) of the body section 121 and extending from the electrode pattern 133 to near the switch 240 through the constricted section 124. The wiring pattern 137 connects the electrode 133 to the ASIC 210.

The antenna pattern 136 is formed at a wiring layer of the flexible substrate 110 at a component mounting surface side (the front side). Meanwhile, the wiring pattern 137 is formed at a wiring layer of the flexible substrate 110 at the back side. This prevents a DC current flowing through the wiring pattern 137 from flowing through the antenna pattern 136 even when, for example, the electrode pattern 133 contacts a charged object and a discharge toward the electrode pattern 133 occurs. Accordingly, the DC current flowing out due to the discharge can be prevented from flowing through the antenna pattern 136 into the SoC 220, and thus, elements in the SoC 220 can be prevented from being electrostatically destroyed. The ASIC 210 includes a protective device against electrostatic discharge at an area where an input circuit to which the wiring pattern 137 is connected is formed. Hereinafter, the antenna pattern 136 is also simply referred to as an antenna 136.

The flexible substrate 110 has a slit 128 extending toward the inside along a direction perpendicular to the longitudinal direction from an edge between the component mounting section 126 and the battery setting section 127 in order to be deformed by receiving stress from the outside.

FIG. 3 is a diagram illustrating a state in which the biosensor 100 of FIG. 1 is affixed to a chest of the subject P. For example, the biosensor 100 is affixed to the subject P with the pad section 123 at an upper side and the pad section 125 at a lower side, with the longitudinal direction of the biosensor 100 extending along a sternum of the subject P. That is, the biosensor 100 is affixed to the subject P with the longer constricted section 124 at the lower side. On the back side of the body section 121 of the biosensor 100, an adhesive tape or an adhesive agent is provided to affix the body section 121 to a body surface of the subject P.

The housing 120 of the biosensor 100, with the body section 121 contained therein, has openings at least at positions corresponding to the electrodes 132, 133. The electrodes 132, 133 exposed from the openings can be adhered to the subject P. The biosensor 100 wirelessly communicates with the operation checking device 310 (FIG. 1) in a state in which the biosensor 100 has been affixed to the subject P, and the electrodes 132 and 133 have been adhered to a body surface of the subject P. The biosensor 100 transmits biological information, such as an electrocardiographic signal obtained from the subject P, to the PC 320 (see FIG. 1) via the operation checking device 310.

Subsequently, based on an electrocardiographic waveform or the like displayed on a display screen of the PC 320, a physician or the like determines that the biosensor 100 has been affixed to a proper position. The biosensor 100 then starts an actual measurement of biological information in response to a recording start command transmitted from the PC 320 via the operation checking device 310 based on an operation of the PC 320 performed by the physician or the like.

The biosensor 100 writes biological information, which has been obtained sequentially from the subject P during the measurement, together with time information, to the flash memory 230. When the switch 240 is depressed during the measurement, and then, the switch 240 is kept continuously in the on state, the biosensor 100 sequentially writes time information (indicating the turned-on state) corresponding to the current time to the flash memory 230. For example, the time information is a time counter or the like that is sequentially updated with the passage of time after the start of the main measurement.

The subject P with the biosensor 100 attached thereto depresses the switch 240 if the subject P feels ill such as a palpitation or shortness of breath. While the subject P is feeling ill, the subject P may depress the switch 240 continuously. After the measurement is completed, the reading device 410 reads biological information, such as electrocardiographic signal data, time counter information accompanied by the biological information, and time counter information indicative of turned-on states of the switch 240, for example, from the flash memory 230 of the biosensor 100.

The reading device 410 (FIG. 1) transfers various information read from the flash memory 230 to the PC 420 (FIG. 1). The PC 420 that receives the various information displays biological information, such as an electrocardiographic waveform, on the display screen, and displays timings when the switch 240 has been depressed. This allows the physician or the like operating the PC 420 to determine whether abnormality is present in an electrocardiographic waveform or the like obtained when the subject P felt ill.

For example, a duration of the measurement is set according to a duration for which the biosensor 100 is operable by power supply from the battery 200. For example, the duration of the measurement may be 24 hours (one day), but may be longer depending on the capacity of the battery 200 and the power consumption of the biosensor 100.

In the present embodiment, since wireless communication is not performed during the measurement (a biological information recording mode, which will be described later), the wireless communication function of the SoC 220 is set to the off state. Therefore, the power consumption of the biosensor 100 can be reduced as compared with the case where the wireless communication function is operated during the measurement.

FIG. 4 is a block diagram illustrating an example of a circuit configuration of the biosensor 100 of FIG. 1. The various elements illustrated in FIG. 4 are mounted on the component mounting section 126 illustrated in FIG. 2, except for the electrodes 132 and 133. In addition to the elements described with reference to FIG. 2, the component mounting section 126 includes a DC/DC converter 10, a power supply switch 12, a DC/DC converter 14, a thermistor 16, filters 18, 20 and a resistor division section 22.

The DC/DC converter 10 uses a power supply voltage VCC1 (for example, 3V) output from the battery 200 to generate a power supply voltage VCC2 lower than the power supply voltage VCC1. The DC/DC converter 10 supplies the generated power supply voltage VCC2 to the power supply terminal of the SoC 220, the LED (G), the LED (R), the power supply switch 12, and the power supply terminal of the ASIC 210.

The SoC 220 always operates by receiving the power supply voltage VCC2 from the DC/DC converter 10 while the battery 200 outputs the power supply voltage VCC1. However, the SoC 220 includes, for example, a deep sleep mode (low power consumption mode) that disconnects the connection between the transistor of the built-in circuit block and the power supply line. Therefore, the SoC 220 does not consume power except for the interrupt function for detecting the pressing of the switch 240 in the deep sleep mode.

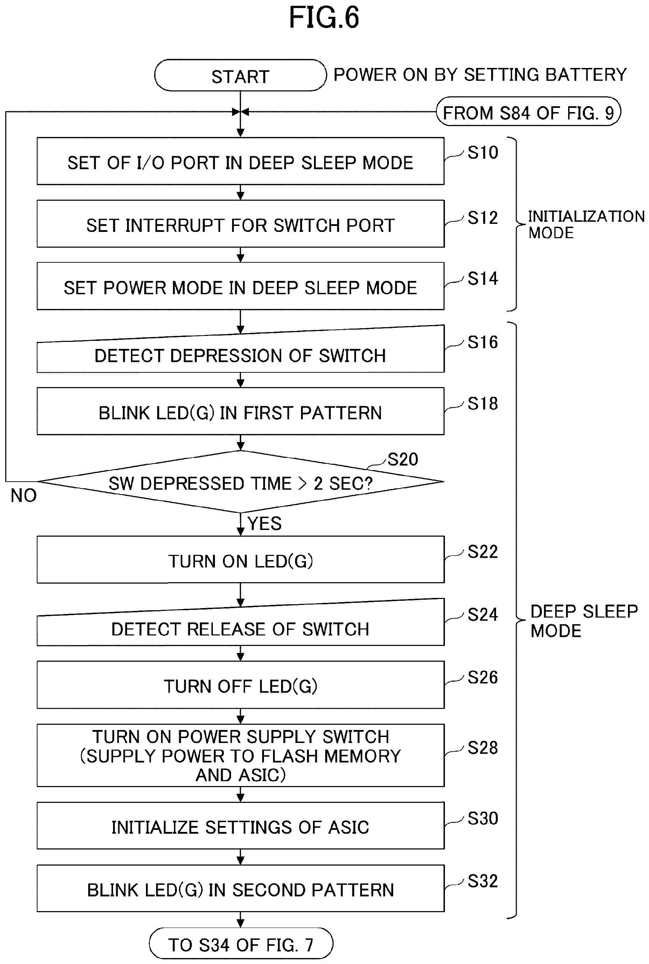

The power supply switch 12 is, for example, a p-channel Metal Oxide Semiconductor (MOS) transistor, and is set to the on state or the off state according to a switch control signal SCNT from the SoC 220. When the power supply switch 12 is set to the on state, the power supply switch 12 connects the power supply line VCC2 to the power supply line VCC2 (S). While the power supply switch 12 is in the on state, the power supply voltage VCC2 is supplied to the flash memory 230 and the thermistor 16 as the power supply voltage VCC2 (S), and is supplied to an enable terminal EN of the DC/DC converter 14. The timing at which the power supply switch 12 is turned on will be described with reference to FIG. 6.

In the deep sleep mode, as described later, the power supply switch 12 is kept in the off state until the switch 240 is depressed for a long time. Therefore, during the deep sleep mode, the DC/DC converter 14, the ASIC 210, and the flash memory 230 stop operating, and no current flows through the thermistor 16. Accordingly, the power consumed by the biosensor 100 in the deep sleep mode can be limited to the power consumed by the SoC 220 in the deep sleep mode and the DC/DC converter 10. By operating only the minimum number of elements, the life of the battery 200 can be extended, so the time during which the biological information can be recorded in the biological information recording mode can be extended.

The DC/DC converter 14 operates while receiving the power supply voltage VCC2 (S) with the enable terminal EN, and uses the power supply voltage VCC1 to generate a power supply voltage VCC3 which is lower than the power supply voltage VCC1. The DC/DC converter 14 stops the generation operation of the power supply voltage VCC3 while the power supply voltage VCC2 (S) is not received with the enable terminal EN. Specifically, the power supply voltage VCC3 is lower than the power supply voltage VCC2, but is not limited to this. The power supply voltage VCC3 is supplied to the ASIC 210. In order to prevent the DC/DC converter 14 from malfunctioning, the enable terminal EN may be connected to a ground wire via a resistive element having a high resistance value that does not affect the power supply voltage VCC2 (S).

The ASIC 210 is connected to the SoC 220 by a Serial Peripheral Interface (SPI, registered trademark). The ASIC receives a master/slave signal M/S of the SPI from the SoC 220. For example, the SoC 220 sets the master/slave signal M/S to a high level when the ASIC 210 is used as a master, and sets the master/slave signal M/S to a low level when the SoC 220 is used as a master.

The ASIC 210 includes an amplifier (hereinafter, AMP) 212, an analog/digital converter (hereinafter, ADC) 214, an input/output interface circuit (hereinafter, I/O) 216, and a logic circuit (hereinafter, LOGIC) 218. Further, the ASIC 210 includes an oscillation circuit (not illustrated) that generates a clock signal CLK, and the generated clock signal CLK is not only used in the ASIC 210 but also output to the SoC 220.

The AMP 212 differentially amplifies voltage signals INP and INN respectively received from the plus electrode 132 and the minus electrode 133 via filters (FLT) 18 and 20 mounted on the flexible substrate 100, and outputs the voltage signals obtained by the amplification to the ADC 214. The ADC 214 converts the differentially amplified voltage signals into a digital value (voltage value), and outputs the voltage value obtained by the conversion to the I/O 216. Then, biological information such as an electrocardiographic signal is output from the I/O 216 to the SoC 220 through the SPI signal line.

The ASIC 210 is operated by receiving the power supply voltages VCC2 and VCC3. For example, the AMP 212, the ADC 214, the LOGIC 218, and the oscillation circuit (not illustrated) are operated by the power supply voltage VCC3.

The SoC 220 includes a Micro Controller Unit (MCU) 222 and a wireless communication section 224. The MCU 222 controls the overall operation of the biosensor 100 by executing a control program stored in the built-in memory. For example, the MCU 222 controls the lighting, blinking, and extinguishing of the LED (G) and the LED (R), and receives the value of the current flowing through the thermistor 16 as temperature information TEMP.

The temperature indicated by the temperature information TEMP indicates the surrounding temperature of the biosensor 100, and when the biosensor 100 is affixed to the subject P, indicates the body temperature of the subject P. Therefore, the body temperature of the subject P can be measured by the biosensor 100. The measured body temperature may be written in the flash memory 230 as biological information together with the electrocardiographic signal and the like.

Further, the MCU 222 detects the value of the power supply voltage VCC1 output from the battery 200 based on a voltage VDET obtained by dividing the power supply voltage VCC1 by the resistor division section 22. As a result, the MCU 222 can notify the decrease in the capacity of the battery 200 to the outside of the biosensor 100 by blinking the LED (R) or the like when the power supply voltage VCC1 is equal to or lower than a predetermined voltage. The MCU 222 may detect a failure of the DC/DC converters 10, 14 or the power supply switch 12 by detecting at least one of the power supply voltages VCC2, VCC2 (S), and VCC3 with the resistor division section (not illustrated).

Also in this case, the MCU 222 can notify the failure to the outside by blinking the LED (R). The MCU 222 may change at least one of the blinking cycle and the blinking pattern of the LED (G) instead of the LED (R) for each type of failure. In this case, the biosensor 100 can notify which element has failed to the outside of the biosensor 100 only by the LED (G) without the LED (R).

Further, the MCU 222 is connected to the switch 240 and can detect whether the switch 240 is pressed. For example, the switch 240 has one end connected to the SoC 220 and the other end connected to the ground wire. Therefore, the MCU 222 can detect the depressing of the switch 240 by the low level of the terminal to which the switch 240 is connected. Note that the terminal of the SoC 220 to which the switch 240 is connected is pulled up. By detecting the depressing time of the switch 240, the MCU 222 can identify multiple events according to the depressing time. That is, a soft switch capable of detecting multiple events can be implemented with a single switch 240.

The SoC 220 is connected to the flash memory 230 by a SPI signal line different from the SPI signal line connected to the ASIC 210. The MCU 222 writes biological information such as an electrocardiographic signal received from the ASIC into the flash memory 230 via the SPI during the biological information recording mode which will be described later.

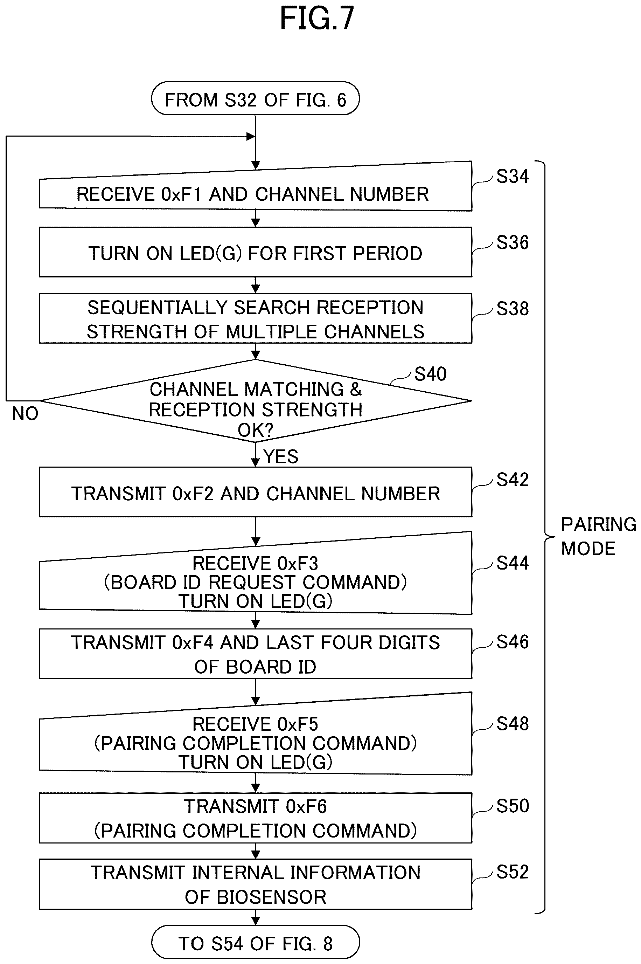

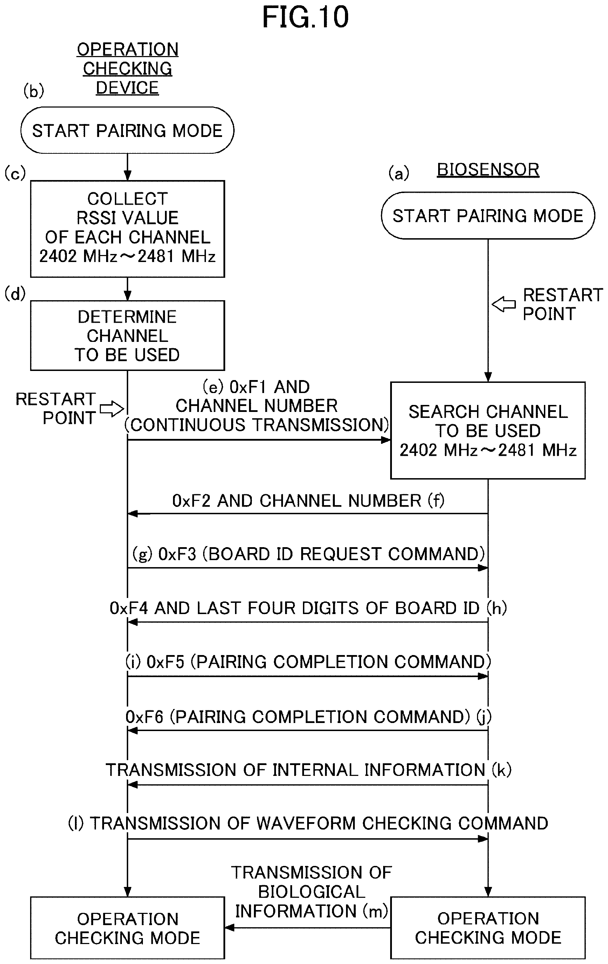

The wireless communication section 224 has a function of communicating with the operation checking device 310 illustrated in FIG. 1 via the antenna 136 based on the instruction from the MCU 222. Further, the wireless communication section 224 has a function of performing pairing with the operation checking device 310 based on the release of the deep sleep mode of the MCU 222. The pairing between the wireless communication section 224 and the operation checking device 310 will be described with reference to FIG. 7 and FIG. 10.

In the biosensor 100 illustrated in FIG. 4, the ASIC 210 functions as an obtaining section for obtaining biological information through the electrodes 132 and 133. The flash memory 230 functions as a memory for storing biological information obtained by the ASIC 210. The SoC 220 functions as a controller including an operation checking mode and a biological information recording mode.

The operation checking mode is a mode of checking as to whether the biosensor 100 can properly obtain biological information (whether the biosensor 100 is properly attached to the subject P and whether the biosensor 100 operates normally). For example, in the operation checking mode, the MCU 222 transmits biological information received from the ASIC 210 to the operation checking device 310 depicted in FIG. 1 via the wireless communication section 224 without writing the biological information to the flash memory 230. Therefore, the power consumption of the biosensor 100 can be reduced as compared with the case where the biological information is written to the flash memory 230 during the operation mode.

The biological information recording mode is a mode of operation switched from the operation checking mode based on a recording start instruction input from the operation checking device 310 when it is checked in the operation checking mode that biological information can be properly obtained by the biosensor 100. During the biological information recording mode, the MCU 222 sequentially writes biological information obtained from the ASIC 210 to the flash memory 230.

FIG. 5 is a state transition diagram illustrating an example of a transition of a mode of operation of the biosensor 100 of FIG. 1. That is, FIG. 5 illustrates an example of an operation control method of the biosensor 100, and illustrates an example of an operation implemented by a control program executed by the biosensor 100.

The MCU 222 transitions to an initialization mode when the battery 200 is set at the battery setting section 127 and generation of the power supply voltage VCC2 is initiated. The MCU 222 performs initial settings of the hardware and the like in the initialization mode. After the completion of initialization, the operation mode transitions to a deep sleep mode. The deep sleep mode is a mode in which the MCU 222 receives an interrupt caused by the switch 240 being depressed, and is a low power consumption mode in which the operation of the ASIC 210 and the wireless communication section 224 is stopped.

In the deep sleep mode, the MCU 222 transitions to a pairing mode when depression of the switch 240 for a long time (for example, two seconds) is detected, and maintains the deep sleep mode in a case where a duration of the switch 240 being depressed is shorter than two seconds. In the deep sleep mode, the MCU 222 transitions to a data output mode when the reading device 410 is connected to the external terminal.

In the pairing mode, the MCU 222 causes the wireless communication section 224 to perform pairing with the operation checking device 310. When the pairing is completed, the MCU 222 transitions to a waiting-for-command mode. If an error occurs during the pairing, the MCU 222 transitions to an error processing mode and performs error processing. The MCU 222 causes the LED (R) to blink in a predetermined pattern (for example, at one second intervals) while the MCU 222 is in the error processing mode.

The MCU 222 transitions from the pairing mode to the deep sleep mode when the pairing mode continues for a predetermined period. For example, the predetermined period is one minute. In the pairing mode, the power of the battery 200 can be prevented from being wasted by transitioning to the deep sleep mode when the state in which pairing is not performed continues.

In the waiting-for-command mode, the MCU 222 transitions to an operation checking mode when a waveform checking command is received from the PC 320 via the operation checking device 310. If an error occurs in the waiting-for-command mode, the MCU 222 transitions to the error processing mode.

In the operation checking mode, the MCU 222 sends an instruction to the ASIC 210 to obtain biological information, and sequentially receives biological information obtained by the ASIC 210. The MCU 222 transmits the received biological information to the PC 320 via the operation checking device 310. When receiving instructions, to stop communication, from the PC 320 via the operation checking device 310 during the operation checking mode, the MCU 222 causes the ASIC 210 to stop obtaining biological information, and returns to the waiting-for-command mode. If an error occurs in the operation checking mode, the MCU 222 transitions to the error processing mode.

In the operation checking mode, when a recording start command is received from the PC 320 via the operation checking device 310, the MCU 222 transitions to a biological information recording mode. Upon the transition from the operation checking mode to the biological information recording mode, obtaining of biological information by the ASIC 210 continues, for example. It is noted that, when the switch 240 is depressed for a long time (for example, ten seconds) in each of the pairing mode, the waiting-for-command mode, and the operation checking mode, the operation mode returns to the deep sleep mode. Further, it is noted that, when the switch 240 is depressed for a long time (for example, ten seconds) in the error processing mode, the operation mode returns to the initialization mode and initial settings are performed in the initialization mode.

The MCU 222 may transition to the deep sleep mode, during the waiting-for-command mode or the operation checking mode, when a sleep request is received from the outside, as well as when the switch 240 is depressed for a long time. For example, the sleep request from the outside is issued from the operation checking device 310 by operating the operation checking device 310 when the operator who operates a biosensor system SYS feels an abnormality during pairing or the like. This enables to prevent the power of the battery 200 from being wasted.

When the operation checking mode continues for a predetermined period, the MCU 222 transitions from the operation checking mode to the deep sleep mode. For example, the predetermined period is 25 minutes. In the operation checking mode, for example, by transitioning to the deep sleep mode when the period in which the biological information is not received continues, the power of the battery 200 can be prevented from being wasted.

Further, the MCU 222 sets a threshold value of the received signal strength in the pairing mode higher than the threshold value used for receiving the signal in the operation checking mode. As a result, the distance between the biosensor 100 capable of wireless communication and the operation checking device 310 can be made shorter than the distance capable of wireless communication in the operation checking mode. For example, the distance between the biosensor 100 capable of wireless communication and the operation checking device 310 is designed to be within 20 cm in the pairing mode and within several meters in the operation checking mode.

As a result, for example, even when pairing multiple biosensors 100 in the same room or the like, a possibility of misconnection with the unintended biosensor 100 can be reduced. In addition, the power consumption during the pairing mode can be reduced. On the other hand, in the operation checking mode after pairing, the distance capable of wireless communication is longer than in the pairing mode, so that wireless communication can be maintained even if the biosensor 100 is apart from the operation checking device 310.

In the biological information recording mode, the MCU 222 sequentially writes biological information received from the ASIC 210 to the flash memory 230. When depression of the switch is detected in the biological information recording mode, the MCU 222 transitions to an event recording mode and then continues to be in the event recording mode until the switch 240 comes to be in a turned-off state. For example, the subject P with the biosensor 100 attached thereto depresses the switch 240 if the subject P feels ill such as a palpitation or shortness of breath. That is, in the event recording mode, information indicating the time when the subject P feels ill such as a palpitation or shortness of breath can be recorded in the flash memory 230 together with the biological information.

When a predetermined set time has elapsed (for example, 24 hours) in the biological information recording mode, the MCU 222 sends an instruction to the ASIC 210 to stop obtaining biological information, and transitions to a waiting-for-data-output mode. In the waiting-for-data-output mode, the MCU 222 waits for the reading device 410 to be connected to the external terminal 131. For example, during the data output mode, the MCU 222 continues to wait for the reading device 410 to be connected to the external terminal 131 by the interrupt terminal. The power consumption while waiting for the interrupt is similar to the power consumption in the deep sleep mode.

The reading device 410 connected to the external terminal 131 via the cable accesses the flash memory 230 through the external terminal 131 to read biological information, time information, and the like stored in the flash memory 230. In each of the waiting-for-data-output mode and the data output mode, if the switch 240 is depressed for a long time (for example, 5 seconds), the operation mode returns to the initialization mode, and initial settings are performed.

In the data output mode, by directly connecting the reading device 410 to the external terminal 131 via the cable, the biological information can be read from the flash memory 230 to the reading device 410 without being controlled by the MCU 222. Because the access to the flash memory 230 by the MCU 222 is not performed and the wireless communication by the wireless communication section 224 is not performed, the power consumption of the biosensor 100 in the data output mode can be minimized.

As illustrated in FIG. 5, the MCU 222 can transition the operation mode to various other operation modes depending on the depressing time of the switch 240 and the operation mode when the switch 240 is pressed. Therefore, as described above, a soft switch capable of detecting a plurality of events can be implemented by one switch 240. As a result, as compared with the case where multiple switches are mounted on the biosensor 100, the biosensor 100 can be miniaturized and the cost of the biosensor 100 can be reduced.

FIG. 6 to FIG. 9 are flow charts illustrating an example of the operation of the biosensor 100 of FIG. 1. The processes illustrated in FIG. 6 to FIG. 9 are implemented by executing the control program by the MCU 222, and correspond to the processes of the state transitions in each operation mode illustrated in FIG. 5. That is, FIG. 6 to FIG. 9 illustrate an example of an operation control method of the biosensor 100. It is assumed that no error occurs in the processes illustrated in FIG. 6 to FIG. 9, and the description of the error processing will be omitted.

When the battery 200 is set on the battery setting section 127 and the generation of the power supply voltage VCC2 is started, the MCU 222 transitions to the initialization mode to execute step S10.

In step S10, the MCU 222 performs setting of the I/O port to be used in the deep sleep mode. Next, in step S12, the MCU 222 sets an interrupt for the switch port to which the switch 240 is connected and the terminal port to which the external terminal 131 is connected. Next, in step S14, the MCU 222 sets a power mode in the deep sleep mode and transitions to the deep sleep mode.

Next, in step S16, the MCU 222 waits until the depression of the switch 240 is detected during the deep sleep mode. When the switch 240 is depressed, the MCU 222 blinks the LED (G) in the first pattern in step S18 to notify the detection of the depression of the switch 240 to the outside. For example, in the first pattern, lighting is repeated at intervals of several tens of milliseconds.

In step S20, the MCU 222 transfers to step S22 when the depressed time of the switch 240 exceeds two seconds. When the depression of the switch 240 is released (off state) before the depressed time exceeds two seconds, the MCU 222 turns off the LED (G) and returns to step S10. The MCU 222 may return to step S16 when the depressed time of the switch 240 is two seconds or less.

In step S22, the MCU 222 turns on the LED (G) to notify the detection of the depression of the switch 240 for a long time to the outside. Next, in step S24, the MCU 222 waits until the depression of the switch 240 is released. When the depression of the switch 240 is released, the MCU 222 turns off the LED (G) in step S26.

Next, in step S28, the MCU 222 sets the power supply switch 12 to the on state, and supplies the power supply voltage VCC2 (S) to the enable terminal EN of the DC/DC converter 14, the flash memory 230, and the thermistor 16. This enables the DC/DC converter 14 to start generating the power supply voltage VCC3, and the ASIC 210 to become enabled for operation.

Next, in step S30, the MCU 222 initialzes settings of the ASIC 210 via the SPI. Next, in step S32, the MCU 222 blinks the LED (G) in the second pattern, and transfers to step S34 of FIG. 7. For example, the blinking of the second pattern of the LED (G) indicates pairing with the operation checking device 310. In the second pattern, lighting is repeated several times per second. After step S32, the operation mode transitions from the deep sleep mode to the pairing mode.

In FIG. 7, the pairing mode process is executed. For example, wireless communication between the wireless communication section 224 and the operation checking device 310 is carried out using the 2.4 GHz band, which is a frequency band for Industrial Scientific and Medical (ISM). In the 2.4 GHz band, any of 80 channels in which 2402 - 2481 MHz is divided into 1 MHz units can be selectively used. Although not particularly limited, the modulation method for wireless communication between the wireless communication section 224 and the operation checking device 310 is, for example, Minimum-Shift Keying (MSK).

In step S34, the MCU 222 waits for the reception of the pairing command "0xF1" transmitted from the operation checking device 310 and a channel number indicating one of the 80 channels in the 2.4 GHz band. In this regard, "0x" at the beginning of the pairing command indicates that the last two digits "F1" are hexadecimal numbers, and are not included in the actual pairing command.

Upon the MCU 222 receiving the pairing command "0xF1" and the channel number, the MCU 222 turns on the LED (G) for the first period in step S36. For example, the lighting time of the LED (G) for the first period is several milliseconds. The lighting of the LED (G) for the first period indicates the reception of various commands transmitted from the operation checking device 310. Next, in step S38, the MCU 222 sequentially searches for multiple channels assigned to each communication band to obtain the reception strength of each channel.

Next, in step S40, if there is a channel that matches the channel number received in step S34 among the searched channels and the reception strength of the matched channel is equal to or higher than the predetermined intensity, the MCU 222 determines this channel as the channel to be used. Then, the MCU 222 transfers to step S42. On the other hand, if none of the searched channels match the channel number received in step S34, or if the reception strength of the matched channel is less than the predetermined strength, the MCU 222 returns to step S34 and waits for the reception of a different channel number.

In step S42, the MCU 222 transmits the pairing command "0xF2" and a channel number indicating the channel determined in step S40 to the operation checking device 310. Next, in step S44, the MCU 222 waits for the reception of the pairing command "0xF3" transmitted from the operation checking device 310. When the pairing command "OxF3" is received, the LED (G) is turned on for the first period. The pairing command "0xF3" is a board ID request command that requests a board ID. The board ID is identification information assigned to each biosensor 100 in advance, and, for example, is stored in a predetermined storage area of the flash memory 230 at the time of manufacturing the biosensor 100.

Upon receiving the pairing command "0xF3", in step S46, the MCU 222 transmits the pairing command "0xF4" and, for example, the last four digits of the board ID to the operation checking device 310. Note that the number of digits and the digit position of the board ID to be transmitted are not limited to the above.

Next, in step S48, the MCU 222 waits for the reception of the pairing completion command "0xF5" transmitted from the operation checking device 310. When the pairing completion command "0xF5" is received, the LED (G) is turned on for the first period. The pairing completion command "0xF5" is an example of the pairing completion notification.

Upon receiving the pairing command "0xF5", in step S50, the MCU 222 transmits the pairing completion command "0xF6" indicating the completion of the pairing to the operation checking device 310. Next, in step S52, the MCU 222 transmits internal information including information such as the current temperature of the biosensor 100 and the power supply voltage in use to the operation checking device 310.

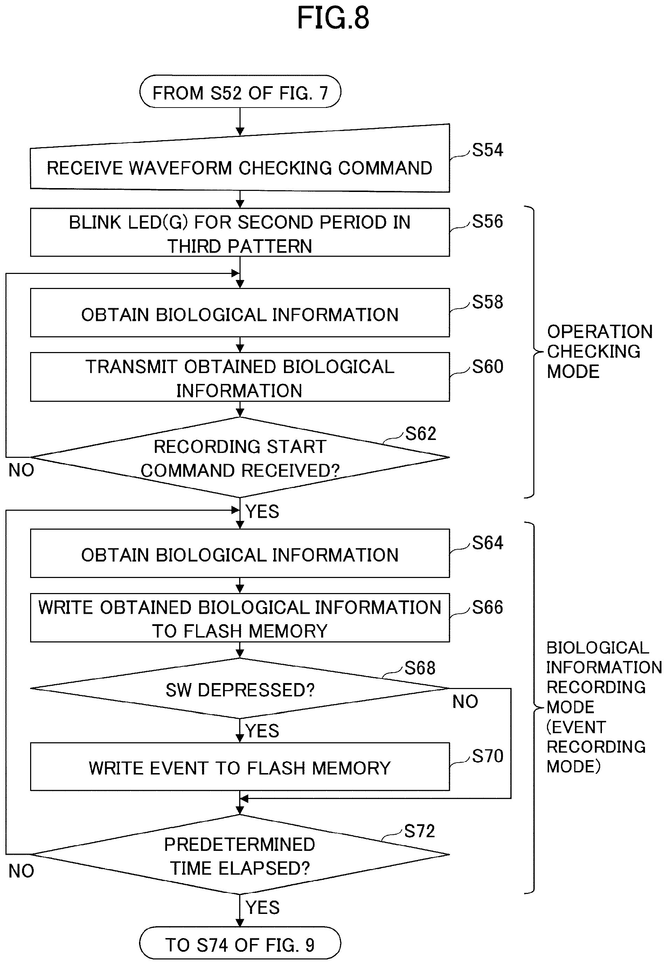

Next, in step S54 of FIG. 8, the MCU 222 waits for the reception of a waveform checking command transmitted from the operation checking device 310. The waveform checking command is issued from the PC 320 when a physician or the like selects a waveform checking button displayed on the screen of the PC 320 in order to check a waveform indicating a time change of the biological information. Upon receiving the waveform checking command, the MCU 222 transitions the operation mode from the pairing mode to the operation checking mode. Then, in step S56, the MCU 222 blinks the LED (G) for a second period in a third pattern in order to notify the outside that the biological information is transmitted to the PC 320. For example, the second period is one minute to several minutes, and the number of blinks per second in the third pattern is different from the number of blinks per second in the second pattern.

Next, in step S58, the MCU 222 causes the ASIC 210 to obtain the biological information, and receives the biological information obtained by the ASIC 210. Next, in step S60, the MCU 222 transmits the received biological information to the operation checking device 310 without writing the received biological information to the flash memory 230.

Next, in step S62, when the MCU 222 receives a recording start command from the operation checking device 310, the process transfers to step S64, and the operation mode transitions from the operation checking mode to the biological information recording mode. If the MCU 222 does not receive the recording start command, the process returns to step S58. That is, the MCU 222 repeatedly executes step S58 and step S60 until the recording start command is received, and transmits the biological information obtained by the ASIC 210 to the PC 320 via the operation checking device 310.

In step S64, during the biological information recording mode, the MCU 222 receives the biological information obtained by the ASIC 210. Next, in step S66, the MCU 222 writes the obtained biological information to the flash memory 230. Writing the biological information to the flash memory 230 is performed during the biological information recording mode, and is not performed during the operation checking mode.

For example, the biological information obtained from the subject P during the operation checking mode is assumed to be written to the flash memory 230. In this case, the MCU 222 is required to delete the biological information written in the flash memory 230 when transitioning from the operation checking mode to the biological information recording mode. In the present embodiment, it is not necessary to delete the biological information written in the flash memory 230 when transitioning from the operation checking mode to the biological information recording mode. Therefore, the storage capacity of the flash memory 230 can be saved, and the capacity of the battery 200 can be saved.

Next, in step S68, the MCU 222 performs step S70 if the depression of the switch 240 is detected, and performs step S72 if the depression of the switch 240 is not detected. Step S70 is the operation of the event recording mode illustrated in FIG. 5, and the MCU 222 writes an event such as time information to the flash memory 230 and transfers to step S72.

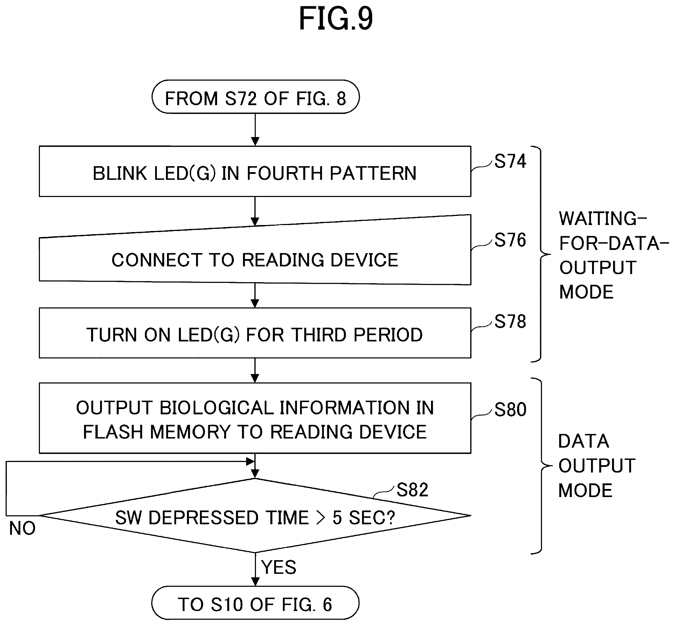

In step S72, the MCU 222 transfers to step S74 of FIG. 9 if a predetermined set time (for example, 24 hours) has elapsed, and returns to step S64 if the set time has not elapsed. That is, the MCU 222 repeatedly performs step S64 to step S70 until the set time elapses. Also, the MCU 222 sequentially writes the biological information obtained by the ASIC 210 to the flash memory 230, or writes an event such as time information to the flash memory. After step S72, the MCU 222 transitions the operation mode from the biological information recording mode to the waiting-for-data-output mode.

In step S74 of FIG. 9, the MCU 222 blinks the LED (G) in a fourth pattern in order to notify the outside that an obtainment period of the biological information has expired. For example, in the fourth pattern, lighting is repeated at intervals of several seconds. Next, in step S76, the MCU 222 waits for the reading device 410 to be connected to the external terminal 131, and when the reading device 410 is connected, performs step S78.

In step S78, the MCU 222 turns on the LED (G) for a third period and transitions the operation mode from the waiting-for-data-output mode to the data output mode in order to notify the outside that the connection to the external terminal 131 of the reading device 410 has been detected. For example, the third period is one to several seconds. Next, in step S80, the flash memory 230 outputs stored biological information or the like to the reading device 410 via the external terminal 131 based on the reading access by the reading device 410. Step S80 is performed while the reading access from the reading device 410 to the flash memory 230 continues.

Next, in step S82, if depression of the switch 240 is detected for five seconds or longer, the MCU 222 returns to step S10 of FIG. 6 and transitions to the initialization mode. The MCU 222 repeatedly performs step S82 if the switch 240 is not depressed for five seconds or longer.

As illustrated in FIG. 6 to FIG. 9, the biosensor 100 changes the lighting time, blinking cycle, or blinking pattern of a single LED (G) according to the depression state of the switch 240 or the internal state of the biosensor 100. This enables, for example, the physician or the like who operates the PC 320 to grasp which operation mode the biosensor 100 is in by observing the lighting state of the LED (G) and to confirm the biosensor 100 is operating correctly.

FIG. 10 is a sequence diagram illustrating an example of operation in a pairing mode of FIG. 5 and FIG. 7. The sequence illustrated in FIG. 10 corresponds to the operation in the operation checking mode of FIG. 8 in addition to the operation in the pairing mode of FIG. 7. Since the operation of the biosensor 100 is the same as the operation of FIG. 7 and FIG. 8, detailed description thereof will be omitted.

The operation checking device 310 is connected to the USB terminal of the PC 320 before the sequence of FIG. 10 is started. Further, the biosensor 100 in transition to the deep sleep mode is attached to the chest of the subject P, and the switch 240 is depressed and held for two seconds or longer, so that the biosensor 100 transitions from the deep sleep mode to the pairing mode (e.g., (a) of FIG. 10). Subsequently, the PC 320 is operated by an operator such as a physician, a pairing command is transmitted from the PC 320 to the operation checking device 310, and the operation checking device 310 starts the pairing mode (e.g., (b) of FIG. 10).

The operation checking device 310 collects the signal strength (for example, Received Signal Strength Indicator (RSSI) value) of each channel in the 2.4 GHz band (e.g., (c) of FIG. 10). The operation checking device 310 determines that the channel having the smallest RSSI value is used for communication with the biosensor 100 as the least used channel (e.g., (d) of FIG. 10). If multiple channels having the smallest RSSI value exist, the operation checking device 310 determines, for example, the channel having the smallest channel number as the channel to be used.

The operation checking device 310 repeatedly transmits the pairing command "0xF1" and the channel number indicating the determined channel together with a synchronization word (not illustrated) (e.g., (e) of FIG. 10). After the pairing mode has been started, the biosensor 100 receives the pairing command "0xF1" and the channel number. Further, the biosensor 100 sequentially searches 80 channels in the 2.4 GHz band to obtain the reception strength of each channel.

If there is a channel whose reception strength is equal to or higher than the predetermined strength of the channel that matches the received channel number, the biosensor 100 transmits the channel number received from the operation checking device 310 together with the pairing command "0xF2" (e.g., (f) of FIG. 10). This enables biological information or the like using a channel whose reception strength is less than the predetermined strength to be prevented from being transmitted/received. Therefore, communication between the biosensor 100 and the operation checking device 310 can be made of a predetermined quality or higher.

If there is no channel that matches the channel number received from the operation checking device 310, or if the reception strength of the channel that matches the channel number is less than the predetermined strength, the biosensor 100 sets the synchronization word to the default value and returns to a restart point. If the pairing command "0xF2" is not received within a predetermined time after the pairing command "0xF1" being transmitted, the operation checking device 310 sets the synchronization word to the default value and returns to the restart point.

Upon receiving the pairing command "0xF2" and the channel number, the operation checking device 310 transmits the pairing command "0xF3" (board ID request command) (e.g., (g) of FIG. 10). When the pairing command "0xF3" is received, the biosensor 100 transmits the pairing command "0xF4" and the last four digits of the board ID (e.g., (h) of FIG. 10).

Upon receiving the pairing command "0xF4" and the part of the board ID, the operation checking device 310 transmits the pairing command "0xF5" (pairing completion command) (e.g., (i) of FIG. 10). When the pairing command "0xF5" is received, the biosensor 100 transmits the pairing command "0xF6" (pairing completion command) (e.g., (j) of FIG. 10). As a result, the pairing between the operation checking device 310 and the biosensor 100 is completed.

By performing pairing with the method illustrated in FIG. 10, communication can be performed between the biosensor 100 and the operation checking device 310 using a channel having a higher reception strength than the others. Therefore, for example, it is possible to increase the possibility of maintaining a predetermined reception strength as compared with the case of frequency hopping in which channels are switched sequentially. As a result, in the operation checking mode, the biological information obtained by the biosensor 100 can be wirelessly transmitted to the operation checking device 310 without making a communication error or the like.

Subsequently, the biosensor 100 transmits internal information including information such as the current temperature of the biosensor 100 and the power supply voltage in use (e.g., (k) of FIG. 10). When the operation checking device 310 receives the internal information, the operation checking device 310 transmits the received internal information to the PC 320. For example, the PC 320 displays the received internal information on the screen. Subsequently, the PC 320 is operated by the physician or the like, the waveform checking button displayed on the screen is selected, and the PC 320 transmits the waveform checking command to the operation checking device 310.

Upon receiving the waveform checking command from the PC 320, the operation checking device 310 transmits the waveform checking command and transitions the operation mode from the pairing mode to the operation checking mode (e.g., (1) of FIG. 10). When the waveform checking command is received, the biosensor 100 transitions the operation mode from the pairing mode to the operation checking mode, and sequentially transmits biological information such as an electrocardiogram signal obtained by the ASIC 210 (e.g., (m) of FIG. 10). The operation checking device 310 transmits the biological information received from the biosensor 100 to the PC 320 during the operation checking mode. Then, the waveform of the biological signal based on the biological information is displayed on the screen of the PC 320.

If the sequence illustrated in FIG. 10 cannot be continued, each of the operation checking device 310 and the biosensor 100 sets the synchronization word to the default value, and returns to the restart point to restart the sequence. For example, as an example in which the sequence cannot be continued, there is a case where the received response is different from the expected value, or a timeout occurs before the expected response is received. Further, after the transition to the pairing mode, for example, if the pairing is not completed within one minute, the biosensor 100 transitions to the deep sleep mode.

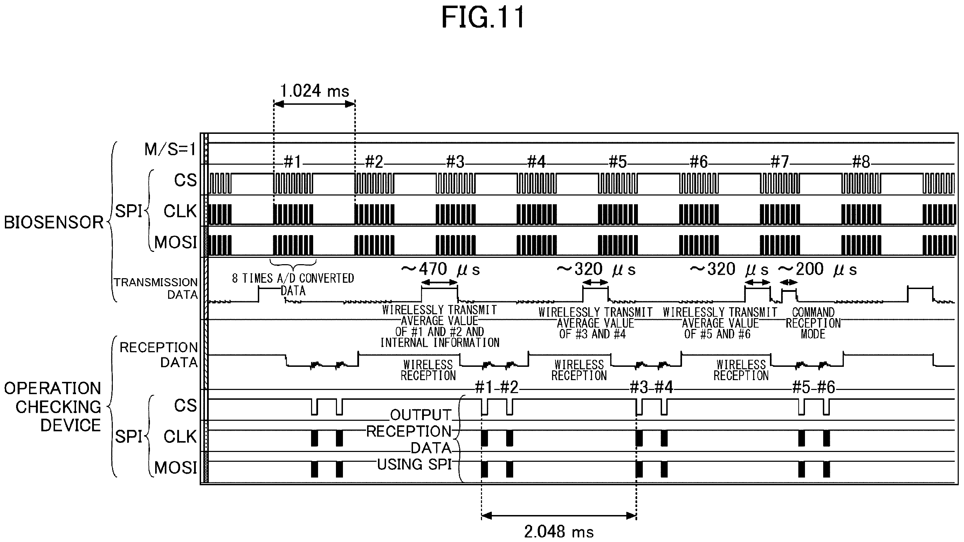

FIG. 11 is a data flow diagram illustrating an example of data transmission/reception between the biosensor of FIG. 1 and the operation checking device 310. In the example illustrated in FIG. 10, the biosensor 100 transmits the biological information obtained from the subject P to the operation checking device 310, and the operation checking device 310 receives the biological information.

In the biosensor 100, when the SoC 220 illustrated in FIG. 4 receives the biological information from the ASIC 210, a master-slave signal M/S is set to the logical value 1 in order to use the ASIC 210 as the master.

The ADC 214 of the ASIC 210 illustrated in FIG. 4 performs A/D conversion of the biological information obtained from the subject P and amplified by the AMP 212 at a predetermined frequency. For example, the ADC 214 performs the A/D conversion eight times every 1.024 milliseconds. That is, the ASIC 210 obtains the biological information eight times every 1.024 milliseconds. In FIG. 11, groups of the biological information obtained eight times every 1.024 milliseconds are indicated by reference numerals #1 to #8.

The I/O 216 uses the SPI to transmit A/D converted biological information to the SoC 220. For example, the I/O 216 sets a chip select CS to the active level (for example, low level) for a predetermined period for each biological information, and outputs the biological information to the data terminal MOSI (i.e., Master Output Slave Input) in synchronization with the clock signal CLK during the active period.

The MCU 222 of the SoC 220 calculates the average value of 16 continuous biological information received from the ASIC 210. That is, the MCU 222 calculates the average value of the biological information included in the two biological information groups (for example, #1 and #2). The MCU 222 outputs the calculated average value to the wireless communication section 224.

The MCU 222 adds internal information to the biological information (e.g., average value) and transmits the average value of the biological information every time the average value of the biological information is transmitted to the operation checking device 310 at a predetermined number of times (for example, any number of times from 10 to 20 times). The wireless communication section 224 transmits the biological information (or the biological information to which the internal information is added) received from the MCU 222 to the operation checking device 310. Although not particularly limited, for example, the time for transmitting the average value of the biological information is up to 320 microseconds, and the maximum time for internal information to be transmitted together with the average value of biological information is 470 microseconds.

When the operation checking device 310 receives the biological information from the biosensor 100, the operation checking device 310 transmits the received biological information as two biological information groups to the data processing section in the operation checking device 310. At this time, the SPI is used for transmission to the data processing section. For example, the operation checking device 310 transmits the biological information (average value) of two biological information groups to the built-in data processing section every 2.048 milliseconds.

The biosensor 100 (MCU 222) transitions to a command reception mode for receiving a control command from the operation checking device 310 at a predetermined frequency. For example, the frequency of transitioning to the command reception mode is set to be the same as the frequency of transmitting the internal information. For example, the operation checking device 310 transmits a stop instruction for transmitting biological information to the biosensor 100 as the control command.

The operation checking device 310 continuously transmits the control command. A transmission interval of the control command is set shorter than the period for transitioning to the command reception mode (for example, up to 200 microseconds). As a result, even when the operation checking device 310 transmits the control command without recognizing the transition timing of the command reception mode, the biosensor 100 can reliably receive the control command.

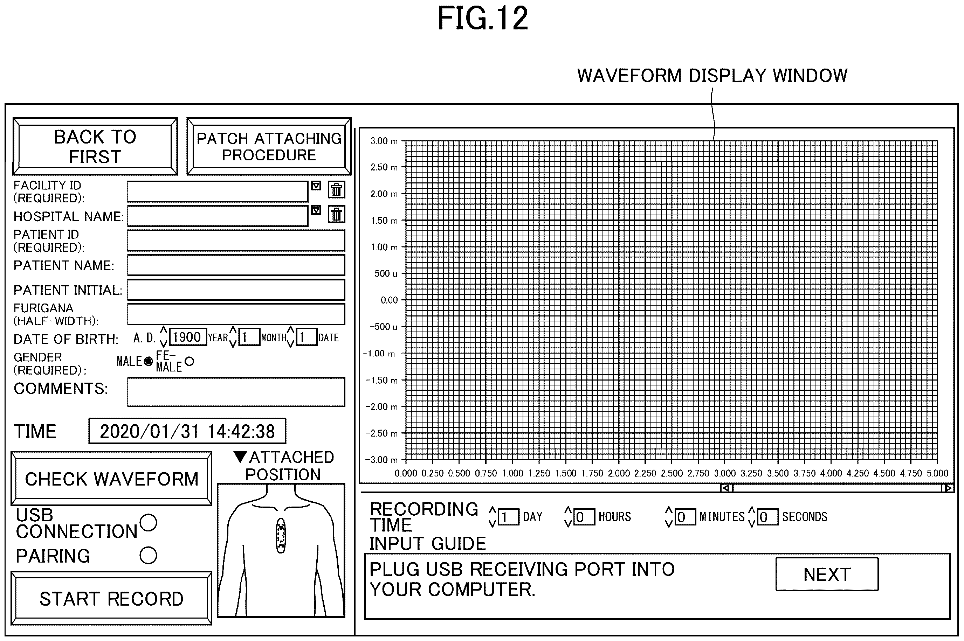

FIG. 12 is an explanatory diagram illustrating a display example of a screen of the PC 320 connected to the operation checking device 310 of FIG. 1. When the operation checking program of the biosensor 100 is started on the PC 320, the operation checking screen illustrated in FIG. 12 is displayed on the screen of the PC 320. The operation checking screen includes an input field for inputting information such as a hospital name, a patient ID, a patient name, and a date of birth, a waveform checking button, a recording start button, and a waveform display window.

For example, an operator such as a physician who operates the biosensor system SYS connects the operation checking device 310 to the PC 320, and after attaching the biosensor 100 to the subject P, the switch 240 of the biosensor 100 is depressed for a long time. By depressing the switch 240 for a long time, the biosensor 100 and the operation checking device 310 perform a pairing process. The operation checking program executed by the PC 320 is started by the operation of the operator before or after the depression of the switch 240 for a long time.

Subsequently, as described above, when the waveform checking button is selected by the operator, the PC 320 transmits an operation checking command to the biosensor 100 via the operation checking device 310. Upon receiving the operation checking command, the biosensor 100 transitions to the operation checking mode and wirelessly transmits the biological information obtained from the subject P to the operation checking device 310. The operation checking device 310 that has received the biological information transmits the received biological information to the PC 320. The PC 320 generates a waveform using the received biological information, and displays the generated waveform in the waveform display window.

The operator checks that the biosensor 100 is affixed to the correct position of the subject P and that the biosensor 100 is operating normally by observing the waveform displayed in the waveform display window. That is, it is confirmed that the biosensor 100 obtains biological information normally.

Subsequently, as described above, when the recording start button is selected by the operator, the PC 320 transmits a recording start command to the biosensor 100 via the operation checking device 310. Upon receiving the recording start command, the biosensor 100 transitions from the operation checking mode to the biological information recording mode, and stops transmitting the biological information obtained from the subject P to the PC 320. Then, the biosensor 100 starts the actual measurement of the biological information, and sequentially writes the biological information obtained by the ASIC 210 to the flash memory 230. Subsequently, the operation checking program is terminated by the physician or the like who operates the PC 420.

For example, after 24 hours have elapsed from the start of writing the biological information to the flash memory due to the start of the biological information recording mode, the reading device 410 (of FIG. 1) is connected to the external terminal 131 of the biosensor 100. The reading device 410 reads the biological information and the like recorded in the flash memory 230 of the biosensor 100 based on the reading instruction from the PC 420, and transfers the read biological information and the like to the PC 420.

For example, the PC 420 displays a waveform (electrocardiogram waveform, etc.) indicating a time change of the received biological information on the screen. When the PC 420 receives accompanying information accompanying the biological information together with the biological information, the PC 420 may display the accompanying information together with the waveform on the screen. Then, the waveform displayed on the screen is observed by the physician or the like who operates the PC 420.

As described above, in the embodiment illustrated in FIG. 1 to FIG. 12, the biosensor 100 transitions to the operation checking mode that enables checking of whether the biological information can be normally obtained before the biological information recording mode for the actual measurement of the biological information. This enables, in the operation confirmation mode, after the physician or the like checks whether the biosensor 100 is correctly attached to the subject P and whether the biosensor 100 operates normally, the biosensor 100 to start the actual measurement of the biological information. As a result, a problem that the biosensor 100 cannot correctly record the biological information during the biological information recording mode can be prevented. Also, a problem that the correct biological information is not written to the flash memory can be prevented.

The biological information obtained during the operation checking mode is wirelessly transmitted to the operation checking device 310 and transferred to the PC 320. Then, by displaying the waveform or the like on the screen of the PC 320, a physician or the like can observe the waveform and check whether the biosensor 100 normally obtains the biological information. Further, after checking that the biological information is normally obtained, the recording start button is pressed by the physician or the like, so that the biosensor 100 transitions from the operation checking mode to the biological information recording mode and the biological information can be written in the flash memory 230. At this time, since the wireless communication section 224 does not operate during the biological information recording mode, the power consumption of the biosensor 100 can be reduced.

As described with reference to FIG. 5, the biosensor 100 can transition the operation mode to various other operation modes according to the depressing time of the switch 240 and the operation mode when the switch 240 is pressed. Therefore, a soft switch capable of detecting a plurality of events can be implemented by one switch 240. Accordingly, the biosensor 100 can be miniaturized and the cost of the biosensor 100 can be reduced.

By performing pairing with the method illustrated in FIG. 10, communication can be performed between the biosensor 100 and the operation checking device 310 using a channel having a higher reception strength than the others. Therefore, in the operation checking mode, the biological information obtained by the biosensor 100 can be wirelessly transmitted to the operation checking device 310 without making a communication error or the like.

The biosensor 100 changes the lighting time, blinking cycle, or blinking pattern of a single LED (G) according to the depression state of the switch 240 or the internal state of the biosensor 100. This enables, for example, the physician or the like who operates the PC 320 to grasp which operation mode the biosensor 100 is in by observing the lighting state of the LED (G) and to check the biosensor 100 being operating correctly.

By directly connecting the external terminal 131 to the flash memory 230, in the data output mode, by connecting the reading device 410 to the external terminal 131 via the cable, the biological information can be read directly from the flash memory 230 to the reading device 410. Since the control by the MCU 222 is not performed and the wireless communication by the wireless communication section 224 is not performed when the biological information is read from the flash memory 230, the power consumption of the biosensor 100 can be minimized.

When the switch 240 is depressed during the biological information recording mode, by writing the time information corresponding to the current time to the flash memory 230, the time when the subject P feels ill such as a palpitation or shortness of breath can be recorded in the flash memory 230 together with the biological information. This enables the physician or the like to determine whether abnormality is present in an electrocardiographic waveform or the like obtained when the subject P felt ill based on such as a depression timing of the switch 240 read from the flash memory 230 and displayed on the screen.

By providing the power supply switch 12 that is kept in the off state until the switch 240 is depressed for a long time during the deep sleep mode, unnecessary power consumption during the deep sleep mode can be prevented. As a result, the life of the battery 200 can be extended, so the time during which the biological information can be recorded in the biological information recording mode can be extended.

Although the above-described embodiment describes an example of using the SPI for data transmission between devices, for example, another serial interface such as an Inter-Integrated Circuit (I2C) may be used.

Although the present invention has been described based on the embodiments, the present invention is not limited to the specifically disclosed embodiments. In these respects, modifications/changes can be made without departing from the spirit of the present invention.

The present application claims priority under Japanese Patent Application No. 2020-059655 filed March 30, 2020. The contents of which are incorporated herein by reference in their entirety.

### [Reference Signs List]

10 DC/DC converter
12 power supply switch
14 DC/DC converter
16 thermistor
18, 20 filters
22 resistor division section
100 biosensor
110 flexible substrate
120 housing
121 body section
122 constricted section
123 pad section
124 constricted section
125 pad section
126 component mounting section
127 battery setting section
127a, 127b pad sections
127c constricted section
128 slit
131 external terminal
132, 133 electrode patterns
134 positive electrode pattern
135 negative electrode pattern
136 antenna pattern
200 battery
210 ASIC
212 amplifier
214 analog/digital converter (ADC)
216 input/output interface circuit (I/O)
218 logic circuit (LOGIC)
220 SoC
230 flash memory
240 switch
250 LED
260 plate member
310 operation checking device
320 PC
410 reading device
420 PC
P Subject
SCNT switch control signal
SYS Living body sensor system
TEMP temperature information
VCC1, VCC2, VCC2 (S), VCC3 supply voltage
VDET voltage

## Claims

1. A biosensor, comprising:
an electrode configured to be attached to a living body;
an obtaining section configured to obtain biological information through the electrode;
a memory configured to store the biological information obtained by the obtaining section; and
a controller including an operation checking mode and a biological information recording mode,
wherein the controller,
during the operation checking mode, wirelessly transmits the biological information obtained by the obtaining section, and transitions to the biological information recording mode upon receiving a recording start command from an outside, and
during the biological information recording mode, writes the biological information obtained by the obtaining section to the memory.

2. The biosensor as claimed in claim 1, further comprising a switch configured to be set to an on state or an off state,
wherein the controller,
includes a sleep mode and a pairing mode that performs pairing with an external device,
during the sleep mode, transitions to the pairing mode based on the on state of the switch being continued for a first predetermined time, and
upon receiving an operation checking command from the external device after the pairing is completed, transitions from the pairing mode to the operation checking mode.

3. The biosensor as claimed in claim 2, wherein the controller sets a threshold value of a reception signal strength in the pairing mode higher than a threshold value used for receiving a signal in the operation checking mode.

4. The biosensor as claimed in claim 2 or 3, wherein, when the pairing mode continues for a predetermined period or when the operation checking mode continues for a predetermined period, the controller transitions to the sleep mode.

5. The biosensor as claimed in any one of claims 2 to 4, wherein, upon receiving a sleep request from the outside during the pairing mode or the operation checking mode, the controller transitions to the sleep mode.

6. The biosensor as claimed in any one of claims 2 to 5, wherein the controller, in the pairing mode,
searches sequentially a plurality of channels assigned to each communication band,
when the searched channel matches a channel indicated by a channel number received from the external device and a reception strength is equal to or higher than a predetermined strength, transmits a channel number of the searched channel to the external device,
after transmitting the channel number to the external device, when a request command requesting identification information of the biosensor is received from the external device, transmits the identification information to the external device,
after transmitting the identification information to the external device, when a pairing completion notification indicating a completion of pairing is received from the external device, transmits a pairing completion response to the external device, and
subsequently, wirelessly communicates with the external device using the channel for which pairing is completed.

7. The biosensor as claimed in any one of claims 2 to 6, further comprising a light emitting element,
wherein the controller,
when the switch is turned on during the sleep mode, sets the light emitting element to a first light emitting state,
when the on state of the switch is continued for a first predetermined time, sets the light emitting element to a second light emitting state, and
when the switch is turned off after the first predetermined time is continued, sets the light emitting element to a third light emitting state and transitions from the sleep mode to the pairing mode.

8. The biosensor as claimed in claim 7, further comprising an external terminal connected to the memory,
wherein the memory outputs the biological information recorded during the biological information recording mode to the external terminal, according to a read request received via the external terminal, and
wherein the controller, when a second predetermined time has elapsed from the transition from the operation checking mode to the biological information recording mode, stops obtaining the biological information by the obtaining section and sets the light emitting element to a fourth light emitting state, and
when a connection of the external device to the external terminal is detected, sets the light emitting element to a fifth light emitting state.

9. The biosensor as claimed in any one of claims 2 to 8, wherein the controller, when the switch is detected to be turned on during the biological information recording mode, sequentially writes time information corresponding to a current time to the memory while the switch is kept in the on state.

10. The biosensor as claimed in any one of claims 2 to 8, further comprising:
a power supply line; and
a power supply switch, provided between a power supply terminal of the obtaining section and a power supply terminal of the memory, configured to be set to an on state or an off state based on a control by the controller,
wherein the controller sets the power supply switch from the off state to the on state when transitioning from the sleep mode to the pairing mode.

11. A biosensor system, comprising:
a biosensor, and
an external device that wirelessly communicates with the biosensor,
wherein the biosensor includes:
an electrode configured to be attached to a living body;
an obtaining section configured to obtain biological information through the electrode;
a memory configured to store the biological information obtained by the obtaining section; and
a controller including an operation checking mode and a biological information recording mode, and
wherein the controller,
during the operation checking mode, wirelessly transmits the biological information obtained by the obtaining section, and transitions to the biological information recording mode upon receiving a recording start command from an outside, and
during the biological information recording mode, writes the biological information obtained by the obtaining section to the memory.

12. The biosensor system as claimed in claim 8, wherein the biosensor further includes a switch configured to be set to an on state or an off state,
wherein
the controller includes a sleep mode and a pairing mode that performs pairing with an external device, and, during the sleep mode, transitions to the pairing mode based on the on state of the switch being continued for a first predetermined time,
the external device transmits a channel number indicating a channel having a lowest signal strength among a plurality of channels assigned to each of a plurality of communication bands,
the controller, in the pairing mode, searches sequentially the plurality of channels, and, when the searched channel matches a channel indicated by a channel number received from the external device and a reception strength is equal to or higher than a predetermined strength, transmits a channel number of the searched channel to the external device,
the external device, upon receiving the channel number, transmits a request command requesting identification information of the biosensor,
the controller, upon receiving the request command from the external device, transmits the identification information to the external device,
the external device, upon receiving the identification information, transmits a pairing completion notification indicating a completion of pairing,
the controller, upon receiving the pairing completion notification from the external device, transmits a pairing completion response to the external device,
subsequently, the external device and the controller wirelessly communicate using the channel for which pairing is completed, and
the controller, after transmitting the pairing completion response, transitions from the pairing mode to the operation checking mode.

13. The biosensor system as claimed in claim 8 or 9, wherein the biosensor further includes an external terminal connected to the memory, and
wherein the external device connected to the external terminal transmits a read request to the memory via the external terminal, and receives the biological information output from the memory according to the read request via the external terminal.

14. A method of controlling an operation of a biosensor, the biosensor including an electrode configured to be attached to a living body, an obtaining section configured to obtain biological information through the electrode, a memory configured to store the biological information obtained by the obtaining section, and a controller configured to control the obtaining section and the memory, the method comprising:
the controller including an operation checking mode and a biological information recording mode,
during the operation checking mode, wirelessly transmitting, by the controller, the biological information obtained by the obtaining section, and transitioning to the biological information recording mode upon receiving a recording start command from an outside, and
during the biological information recording mode, writing, by the controller, the biological information obtained by the obtaining section to the memory.
